# EUROPEAN PATENT APPLICATION

(11) **EP 3 967 294 A1**
(43) Date of publication of application: **16.03.2022**
(21) Application number: 20214121.4
(22) Date of filing: 15.12.2020
(51) Int. Cl.: A61K 8/67, A61Q 19/08, A61K 8/37, A61P 17/02, A61Q 17/02, A61K 31/355, A61K 31/215, A61K 31/265, A61K 31/08, A61K 47/08, A61K 47/12, A61K 47/14, A61Q 19/00, A61K 8/36

(54) **DERMATO-COSMETIC COMPOSITION FOR TOPICAL USE**

(30) Priority: 09.09.2020 IT 202000021370
(71) Applicant: Sigla S.r.l., 29121 Piacenza (IT)
(72) Inventor: Facchini, Gabriele, 29121 Piacenza (IT)
(74) Representative: Fisauli, Beatrice A. M.

(57) **Abstract**

The invention relates to an anhydrous and biodegradable composition comprising natural vitamin, and/or its derivatives and/or mixtures thereof, and a carrier component V, anhydrous, lipophilic.

The composition is indicated for moisturising purposes and for skin problems.

## Description

The present invention relates to a composition based on a fat-soluble active principle, in particular vitamin E and/or its derivatives, and one or more oilbased carriers, said components being of completely natural origin, along with the topical use of said composition, in particular for dermo-cosmetic purposes. More specifically, the present composition is indicated for non-therapeutic use, in the presence of a skin problem, such as, for example, fissures, cuts and grazes, etc.

Known compositions based on vitamin E that are already commercially available are compositions that include components not of natural origin, such as silicones and petroleum jelly, as carriers for the vitamin E.

However, the use of these components not of natural origin causes problems, primarily because these components are not biodegradable. Following recent increases in environmental awareness, it has been realised that the lack of biodegradability of these components involves, over time, their accumulation in the environment, land and sea, causing pollution and, therefore, harm to the entire planetary ecosystem.

This invention aims to overcome the above mentioned problems with prior art compositions in which components not of natural origin are used as carrier agent.

It has now surprisingly been found that a composition based on natural vitamin E, as specified in the following, is highly active, more so than similar products currently present on the market.

Although this invention is not restricted by any particular theory on the action mechanism (and therefore bound by it), it is considered that the greater activity shown by the composition according to the present invention is due to the presence of natural vitamin E, in a free form or as a derivative, which is more bio-available, indicatively at least twice as much, as vitamin E of a synthetic type.

Furthermore, as the active principle used in said composition is of totally natural origin, more specifically of plant origin, and as the carrier component is also of completely plant origin, the composition as a whole has the intrinsic advantage of being completely plant-based and, therefore, biodegradable and ecologically sustainable.

Within this invention, the expression "natural origin" is understood to mean that the chemical component has not been obtained by synthesis in a laboratory, whether industrial or other.

The term "plant origin", on the other hand, is understood to mean that the chemical component derives from plant substances subjected to treatments such as extraction, distillation, pressing, fractionation, purification, concentration or fermentation.

Finally, advantageously, said composition shows slow absorption by the skin, which encourages it to carry out its activity, as described in the following, on the composition application site.

Object of the present invention is therefore an anhydrous composition C, comprising (percentages by weight):
I) 30-70%, preferably 40-60%, more preferably 45-55%, of an active principle selected from vitamin E in the form of R,R,R-d-α-tocopherol isomer, its derivatives and mixtures thereof, and
II) 30-70%, preferably 40-60%, more preferably 45-55%, of carrier component V, anhydrous and lipophilic.

Said component V comprising one or more of the following lipophilic compounds:
alkyl esters (EA) obtained by reaction of:
(1) an inorganic acid, such as a C₈-C₂₆ fatty acid, saturated or monounsaturated or polyunsaturated, and
(2) an inorganic acid, such as carbonic acid,
   with an alcohol chosen from among:
   i) monohydroxylic aliphatic alcohols, of formula R-OH, where R is an alkyl radical C₄-C₂₆, preferably C₆-C₂₄, straight or branched, saturated or unsaturated, and
   ii) polyhydroxylic aliphatic alcohols C₁-C₆, preferably with two or three hydroxyl groups, such as 1,2-propandiol and glycerol.

Said fatty acids may be mono- or, even, pluricarboxylic, generally dicarboxylic, at least one of the carboxylic groups will be esterified. In the case of pluricarboxylic fatty acids, the carboxylic groups may be esterified with the same alcohols or with different alcohols.

Specific preferred examples of suitable compounds are the esters of fatty acids selected from: arachidic acid, docosanoic acid (or behenic acid), erucic, lignoceric, oleic, ricinoleic, stearic, linoleic, alpha-licanic, alpha-eleostearic, alpha-linolenic, palmitic, tetradecanoic (or myristic acid), dodecanoic (or lauric acid), decanoic (or capric acid), caprylic acid.

More specifically said compounds are selected, for example, from propyl-heptyl caprylate, caprylyl caprylate/caprate, dicaprylyl carbonate, coco caprylate, coco caprylate/caprate, propylene glycol dicaprylate dicaprate, ethylhexyl palmitate, ethylhexyl stearate, hexyl laurate, dibutyl adipate, isopropryl palmitate or myristate, cetearyl ethyl hexanoate.

Naturally, the component V may also consists of two or more of said compounds. In that case, said compounds may be present in any ratio by weight.

As is known to the art, the following above mentioned terms are used to designate the following mixtures of esters:
- "caprylyl caprylate/caprate": mixture of esters of caprylic and capric acids with caprylic alcohol;
- "coco caprylate": mixture of esters of capric acid with coconut fatty alcohols;
- "coco caprylate/caprate": mixture of esters of caprylic and capric acids with coconut fatty alcohols; and
- "propylene glycol dicaprylate/dicaprate": a mixture of diesters of propylene glycol with caprylic acid and capric acid.

In the composition according to the present invention, component V is typically of natural origin, preferably plant. Said component V is totally biodegradable. The natural origin index of component V is defined under guideline ISO 16128. As mentioned previously, component V acts as a carrier means for the at least one active principle present in composition C. As well as this function, component V generally also has other actions, such as, for example, moisturising, soothing, nourishing, softening, protecting.

If component B is an ester, this is preferably one without smell or with only a slight fragrance.

Component V preferably also comprises, along with said alkyl esters (EA), one or more additional components.

Component V preferably also comprises, along with said alkyl esters, one or more additional components selected from waxy substances and plant oils, the latter if necessary partially or fully hydrogenated.

More specifically, said additional components are selected, preferably, from substances such as
- alcohols, in particular fatty alcohols,
- plant oils, the latter if necessary partially or fully hydrogenated.

A particular example of fatty alcohols is docosanol (or behenyl alcohol).

Said plant oil may be, for example, the oil known as Olus Oil (CAS number: 68956-68-3).

According to a preferred embodiment, composition C according to the present invention comprises the following components (percentages by weight):
- 30-70% of an active principle selected from vitamin in the form of R,R,R-d-α-tocopherol isomer, and mixtures thereof,
- 5-20% of said alkyl ester (EA),
- 15-35% of said plant oil, and
- 5-20% of said fatty alcohol.

In said embodiment, preferably, said composition comprises from 10 to 25% by weight of Olus Oil.

In this description said plant oils can also be understood to refer to the oils commonly used in cosmetics, such as avocado oil, coconut oil, jujube oil, sesame oil, etc. or mixtures thereof. Said oils are well known to an expert in the field.

Component V is produced using procedures known to an expert in the field, so these will not be described in the present description.

According to a preferred embodiment, if in the composition according to the present invention component V is in the form of certain esters, such as caprylyl caprylate/caprate, these are for the most part, that is to say at least 75%, preferably at least 80%, more preferably at least 85%, by weight, or even entirely, produced by means of an eco-sustainable procedure Thus, typically, only a reduced fraction of said component V in the form of an ester, that is to say 25% or less, generally between 0.5 and 20%, in particular between 1 and 15%, preferably between 1 and 10%, by weight, is produced using a procedure that does not implement fermentation.

Said fermentation procedure, implemented according to known technology, comprises the use of micro-organisms and/or enzymes. The latter derive, preferably, from natural organisms, such as plants and yeasts. A first advantage of the procedures involving the use of enzymes is, in this specific case, the efficiency of these methods as a result of the high selectivity of the enzymes that allow very uniform production, that is to say with a very low rate of sub-products. Sub-products also refer to a product with a different stereospecificity from the one required.

Fermentation procedures also have the advantage of producing less processing waste compared to traditional procedures, as the micro-organisms and/or enzymes can be used repeatedly in the subsequent procedures to obtain the same types of product, so that the environmental impact is reduced with respect to a conventional process, in which the chemical catalysts, which are used in place of the enzymes, are generally eliminated after having been used once only.

Preferably, the enzymes that are used as biocatalysts in the procedures to produce said component V are certified as produced by non-genetically modified micro-organisms.

In the composition according to the present invention, as mentioned previously, the vitamin E (or tocopherol) is of natural origin, so the vitamin E is only present as a R,R,R-α-tocopherol isomer, also known as d-α-tocopherol or 2R,4'R,8'R-α-tocopherol, instead of in the form of a raceme.

In the present description, unless specified, "vitamin E2 is intended to refer both to vitamin E in its free form and its derivatives and mixtures thereof.

Vitamin E derivatives are, typically, its ethers, its esters and their physiologically acceptable salts.

It is known that vitamin E and its derivatives obtained from natural-plant sources have a much higher bioavailability than vitamin E and its derivatives of synthetic origin.

Should the composition C according to the present invention contain two or more compounds that can be traced to vitamin E, said compounds may be present in any ratio by weight.

Vitamin E in the form of an ester is esterified with an organic acid, generally, with a low number of carbon atoms, typically C₂-C₈, typically mono- or dicarboxylic, generally acetic or succinic acid.

The vitamin E, and its derivatives, used in the composition of the present invention can be obtained, typically, by distillation of plant oils.

Optionally, the composition C may comprise, in addition to the above mentioned components, at least an additional active component (or active principle), component (III). Naturally, said component (III) is likewise of natural origin, preferably of plant origin, in any case totally biodegradable.

In particular, said additional active component is, preferably, at least in part fat-soluble, more preferably it is totally fat-soluble. This component is selected, for preference, from those capable of being in synergy with the activity of vitamin E and/or its derivatives, such as, merely as an example, polyunsaturated fatty acids, in particular one or more of ω-3-, ω-6- and ω-9-polyunsaturated C₁₀-C₂₂ fatty acids and the like, and mixtures thereof.

Suitable ω-3-polyunsaturated fatty acids are, for example, eicosapentaenoic acid (EPA), docosahexaenoic acid (DHA), docosapentaenoic acid (DPA) and alpha-linolenic acid (ALA).

Examples of further suitable fatty acids, in particular ω-6- and/or ω-9-polyunsaturated, are linoleic acid, γ-linolenic acid, arachidonic acid and docosadienoic acid, to mention just a few.

If present, said additional active component is in an amount between 0.5 and 20%, preferably between 1 and 15%, more preferably between 2 and 10%, by weight with respect to the entire weight of composition C.

Naturally, "additional active component" is understood to refer also to two or more active components. In this case, said active components can be in any ratio by weight with each other.

Following the components present in composition C according to the present invention, said composition C is therefore, at least to a great extent, that is to say equal to or exceeding 95%, preferably 100%, by weight, of plant origin and, therefore, totally biodegradable. More specifically, said composition C, like its components (II) and (III), have a natural origin index of between 0.7 and 1, more preferably between 0.9 and 1.

To assess the ecological profile of the composition according to the present invention, the term "natural origin index" is used here to refer to the percentage by weight of the component directly obtained from natural sources or obtained from immediate predecessors that can be obtained directly from natural sources.

According to current regulations, an ingredient with a natural origin index equal to 1 is considered natural, whereas an ingredient with a natural origin index of 0.5 or above, but less than 1 is a natural derivative.

In certain embodiments, the composition described may include at least one component with an index of naturalness less than 1.

Composition C according to the present invention, therefore, is essentially free from alkane type hydrocarbons in free form.

Composition C according to the present invention can be prepared, typically, using a simple preparation process, a process essentially to mix the two or more components that make it up, using known methods that, consequently, are known to an expert in the field.

As the preparation methods are in themselves known to an expert in the field, they will not be described in detail.

According to a preferred embodiment of this mixing process, the process comprises a mixing stage, that envisages, in a first phase, the mixing together of all the components, whether these be solid or liquid, that are foreseen, with the exception of the vitamin E. This first phase is carried out with heating up to 80-85° C. This first phase extends over a period of time that, generally speaking, is comprised between 10 and 30 minutes.

At the end of this phase there is a second mixing phase, in which the mixture prepared in the first phase is mixed with the vitamin E. This second phase is carried out with heating, preferably, not exceeding 80° C. This second phase has a duration at said temperature not exceeding 10, preferably 8, typically between 3 and 6 minutes.

At the end of the mixing stage, the resulting mixture is cooled to room temperature (approximately 25° C) in a short time. This cooling stage is carried out while keeping the mixture being cooled under stirring. Cooling can take place in various ways, for example by circulating very cold water in the outer sheathing of the mixing container.

Specifically, in said stage the mixture is cooled with an indicative cooling speed of 8-11° C/2 minutes so as to obtain a mixture with a temperature of approximately 35-40°C (first cooling phase). When the mixture has reached this temperature, cooling is continued for a further period of time (second cooling phase), indicatively equal to 7-15 minutes, during which time the stirring speed of the mixture is higher than during the first cooling phase, bringing it, for example, to at least 1.2, preferably 1.5, more preferably 2 times higher than the mixing speed during the first cooling phase.

At the end of this stage, composition C according to the present invention is obtained in the form of a compact and homogeneous mass that retains its consistency both over time, that is to say even various months after its preparation, and following changes in temperature, both in the sense of an increase and of a decrease in the temperature of the composition.

The composition C according to the present invention is suitable for topical use. In particular the composition is suitable for application to the skin as a skin regenerating and nourishing agent. More specifically, said composition is useful both as a moisturiser, especially in the case of dehydration and stress of the nipple and areola (breast-feeding), chapped lips, dry skin around the eyes, and to assist in the treatment of fissures, particularly but not solely anal fissures, and perianal irritation.

Composition C is formulated in different ways, typically so as to appear, for example, in the form of an ointment, cream or gel. These formulations have an anhydrous form.

Composition C according to the present invention is, typically, odourless, generally also colourless and, usually, tasteless.

According to the type of formulation, composition C according to the present invention will be suitable for a specific container, such as a tube, pot, etc., with preference being given to highly eco-sustainable materials.

The following is a non-limiting example illustrating the present invention.

### Materials and methods

The caprylyl caprylate/caprate is produced using a fermentation procedure.

### Example 1

A composition is prepared having the following components (percentages by weight):
1) 15% caprylyl caprylate/caprate
2) 25% plant oil
3) 10% behenyl alcohol, and
4) 50% d-alpha tocopheryl acetate (natural vitamin E)

Said plant oil consists of 80% by weight of Olus Oil and 20% by weight of hydrogenated plant oil.

Components 1) to 3) are placed in a recipient with a cooling casing and mixed for 20 minutes at 81° C, then component 4) is introduced and mixing continues at a temperature of 75° C for 5 minutes.

At the end of the mixing process, the mixture is cooled as indicated above, until the mixture is at room temperature.

The resulting composition takes the form of a compact and homogeneous mass that retains its consistency unchanged six months after its preparation, even when subjected to tests in which the temperature of the composition is increased and decreased.

## Claims

1. An anhydrous composition C, comprising (percentages by weight):
- 30-70% of an active principle selected from vitamin E in the form of R,R,R-d-α-tocopherol isomer, and derivatives thereof, and mixtures thereof;
- 30-70% of carrier component V, anhydrous, comprising one or more of the following lipophilic compounds:
alkyl esters obtained by reaction of:
(1) an inorganic acid, such as a C₈-C₂₆ fatty acid, saturated or monounsaturated or polyunsaturated, and
(2) an inorganic acid, such as carbonic acid,
with an alcohol chosen from among:
i) monohydroxylic aliphatic alcohols, of formula R-OH, where R is an alkyl radical C₄-C₂₆, preferably C₆-C₂₄, straight or branched, saturated or unsaturated, and
ii) polyhydroxylic aliphatic alcohols C₁-C₆, preferably with two or three hydroxyl groups.

2. Composition C according to the preceding claim, in which carrier component V is selected from the esters of caprylic, capric, palmitic, stearic, lauric, adipic, myristic acids or a mixture thereof.

3. Composition C according to any one of the preceding claims, in which component V comprises caprylyl caprylate/caprate and one or more of the additional components selected from hydrogenated or non-hydrogenated vegetable oils and fatty alcohols.

4. Composition C according to any one of the preceding claims, comprising (percentages by weight):
- 40-60% of vitamin E and/or derivatives thereof or their mixtures, and
- 40-60% of component V.

5. Composition C according to any one of the preceding claims, comprising (percentages by weight):
- 45-55 % of vitamin E and/or derivatives thereof or their mixtures, and
- 45-55% of component V.

6. Composition C according to any one of the preceding claims, comprising a component (III) in a quantity of from 0.5 to 20% by weight with respect to the entire weight of composition C, said component (III) being selected from polyunsaturated fatty acids selected from fatty acids ω-3-, ω-6- and ω-9-polyunsaturated C₁₀-C₂₂ or mixtures thereof.

7. A process for the production of composition C according to any one of the preceding claims, comprising a mixing stage and a subsequent cooling stage to room temperature, in which said mixing stage comprises at least two mixing phases, and the vitamin E or its derivatives or mixtures thereof are added to the mixture coming from the preceding phase only during the final mixing phase, said final mixing phase being carried out at a temperature not exceeding 80°C, and said cooling stage being carried out with a cooling speed of 8-11°C/2 minutes until obtaining a mixture with a temperature of approximately 35-40°C (first cooling phase), followed by a second cooling phase for a further period of time of 7-15 minutes, during which the stirring speed of the mixture is at least 1,2 times higher than that during the first cooling phase.

8. Composition C according to any one of the preceding claims 1 to 6 for topical use to treat the skin for at least one of a moisturising and adjuvant treatment for perianal fissures and irritation, cuts and grazes.

9. Composition C according to the preceding claim, to treat nipple and areola dehydration and stress (breast feeding), chapped lips, dry skin around the eyes.
